# FASCICULE DE BREVET EUROPEEN

(11) **EP 2 032 515 B1**
(45) Date de publication et mention de la délivrance du brevet: **29.03.2017**
(21) Numéro de dépôt: 07803789.2
(22) Date de dépôt: 27.06.2007
(51) Int. Cl.: C07C 39/08, C07C 37/84

(54) **NOUVELLE FORME D'HYPROQUINONE ET SON PROCEDE D'OBTENTION**
NEUARTIGE FORM VON HYDROCHINON UND VERFAHREN ZU DESSEN ERHALT
NEW FORM OF HYDROQUINONE AND PROCESS FOR OBTAINING IT

(30) Priorité: 29.06.2006 FR 0605868
(43) Date de publication de la demande: 11.03.2009
(73) Titulaire: Rhodia Opérations, 75009 Paris (FR)
(72) Inventeur: LE THIESSE, Jean-Claude, F-42100 Saint-Etienne (FR)
(74) Mandataire: Menville, Laure
(86) Numéro de dépôt international: PCT/FR2007/001080
(87) Numéro de publication internationale: WO 2008/000956

(56) Documents cités:
- EP-A- 0 740 954
- FR-A1- 2 846 324
- GB-A- 1 275 232
- JP-A- 2000 302 716
- US-A- 3 234 307

## Description

La présente invention a pour objet une nouvelle forme d'hydroquinone.

Plus précisément, l'invention a pour objet des écailles d'hydroquinone.

L'invention se rapporte aussi à la préparation desdites écailles.

L'hydroquinone est un produit largement utilisé dans de nombreux domaines d'application en tant qu'inhibiteur de polymérisation ou anti-oxydant dans les élastomères. Un autre domaine d'application est la photographie. Il s'ensuit que c'est un produit de grande consommation.

L'hydroquinone est actuellement disponible sur le marché sous la forme d'une poudre formée d'aiguilles petites et cassantes. Les inconvénients qui en résultent sont la présence de fines qui entraînent des problèmes de poussiérage lors du stockage et de la manipulation de ladite poudre.

Or les poussières d'hydroquinone ne sont pas sans danger par rapport à l'environnement en raison de risques d'explosion et eu égard à l'Homme car cette substance est irritante pour les yeux, les voies respiratoires et peut également provoquer des irritations de la peau lorsqu'elle est mise à son contact.

On a proposé selon JP-A-2002-302716 de formuler l'hydroquinone sous forme de granulés selon une technique de granulation qui consiste à faire passer la poudre d'hydroquinone entre deux rouleaux permettant d'obtenir des tablettes puis de concasser ces tablettes de façon à obtenir lesdits granulés.

L'hydroquinone sous forme d'écailles a déjà été produit dans GB 1.275.232, mais avec une densité très faible et très poussiérante.

Une autre mise en forme décrite dans EP-A 1556322 consiste à mettre l'hydroquinone sous forme de perles c'est-à-dire des particules solides à forte sphéricité qui sont exemptes de poussières et qui ont une forme physique qui leur confère une bonne résistance à l'attrition.

La taille des particules exprimée par le diamètre médian (d₅₀) est compris entre 300 µm et 2 000 µm, de préférence entre 500 µm et 1500 µm. On définit le diamètre médian comme étant tel que 50 % en masse des particules ont un diamètre supérieur ou inférieur au diamètre médian.

Le procédé de préparation desdites perles consiste à préparer à chaud, une solution aqueuse concentrée d'hydroquinone, puis de fragmenter la solution en gouttelettes par passage au travers d'une buse et de refroidir les gouttelettes obtenues dans un courant gazeux de telle sorte qu'elles se solidifient en perles qui sont ensuite récupérées et séchées.

Ledit procédé relève de la technique de prilling mais contrairement à ce qui est habituellement mis en oeuvre, il ne consiste pas à faire fondre l'hydroquinone et à la fragmenter ensuite par passage dans une buse.

En effet, la difficulté à laquelle était confronté l'Homme du Métier était que l'hydroquinone fond à haute température de 172°C et par ailleurs, l'hydroquinone a une tension de vapeur très élevée (supérieure à 25 mbar à cette température) ce qui entraîne à la sortie de buse, une vaporisation phénoménale entraînant des problèmes de poussières et d'assainissement, rédhibitoires d'un point de vue industrielle.

Ainsi, le procédé proposé par EP-A 1556322 est de préparer des perles d'hydroquinone selon la technique de prilling en partant d'une solution aqueuse d'hydroquinone.

Les perles obtenus selon EP-A 1556322 sont des produits de haute qualité en termes d'absence de poussières et de coulabilité.

L'inconvénient de ce procédé est lié au prilling qui nécessite toujours un investissement élevé au niveau de la capacité et qui implique ensuite une étape additionnelle de séchage des perles obtenues.

L'objectif de la présente invention est de fournir une nouvelle forme ou une nouvelle présentation de l'hydroquinone permettant de pallier les inconvénients précités.

La présente revendication se rapporte à une hydroquinone sous forme d'écailles, particules massives caractérisées par une forme plaquettaire de longueur variant entre 0,5 et 6cm, de largeur variant entre 0,5 et 3cm, d'épaisseur variant entre 400 et 1500 micromètres, présentant une masse volumique apparente (non tassée) d'au moins 0,4 g/cm3 et dont le pourcentage en masse de particules de dimension inférieure à 100 micromètres est inférieur à 3%.

Les particules plaquettaires répondent à un facteur de forme générale défini en plan par un contour très varié qui peut être plus ou moins carré, rectangulaire, rond ou ovale.

Les différentes écailles de forme variée s'inscrivent dans un parallélépipède présentant les dimensions précisées ci-après.

La longueur varie de préférence entre 1 et 3 cm.

La largeur quant à elle, s'échelonne de préférence entre 0,5 et 1,5 cm.

Les mesures sont effectuées sur un échantillon de 20 écailles prélevées au hasard.

Les longueur et largeur sont déterminées par mesure à l'aide d'une règle graduée.

Les parallélépipèdes précités possèdent l'une de leurs trois dimensions (l'épaisseur) beaucoup plus faible que les deux autres (largeur et longueur).

Pour ce qui est de l'épaisseur, elle se situe de préférence entre 500 et 750 µm.

L'épaisseur est mesurée à l'aide d'un pied à coulisse ou d'un appareil Palmer.

La figure 1 représente une photographie prise à l'aide d'un appareil photo numérique qui montre la morphologie de type écaille de l'hydroquinone obtenue selon l'invention.

Il est à souligner que ces particules massives présentent des arrêtes franches.

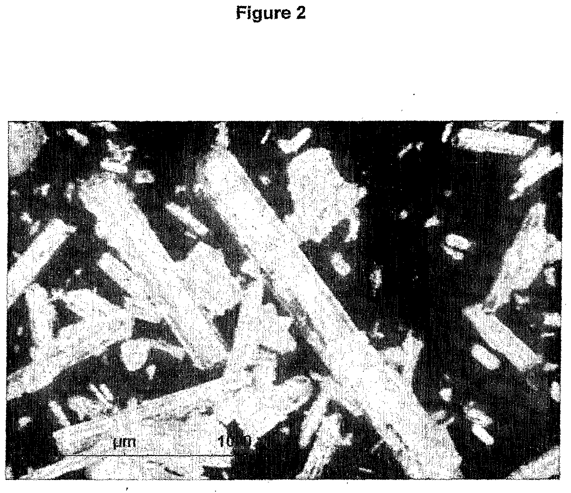

La figure 2 représente une photographie prise à l'aide d'un appareil photo numérique qui montre la morphologie en aiguilles des cristaux de la poudre d'hydroquinone disponible dans le commerce.

La figure 4 représente une photographie prise également à l'aide d'un appareil photo numérique qui montre une meilleure vue d'ensemble du produit de l'invention grâce à un grossissement plus petit et indiqué sur la photographie.

Une caractéristique du produit de l'invention est un taux de particules fines très faible par rapport à une présentation sous forme de poudre.

Le taux des particules fines est défini comme le pourcentage en masse des particules de dimensions inférieures à 100 µm.

Sont considérées selon l'invention, comme particules fines, des particules qui passent à travers un tamis ayant une maille de 100 µm.

Le taux des particules fines est inférieur à 3 % en masse, de préférence compris entre 0,7 et 1,5 % en masse, et plus préférentiellement compris entre 0,7 et 1 %.

On précisera à titre indicatif que la taille des particules fines s'échelonnent entre 1 µm et 100 µm, avec un diamètre médian situé entre 20 et 30 µm.

On définit le diamètre médian comme étant tel que 50 % en masse des particules ont un diamètre supérieur ou inférieur au diamètre médian.

A titre de comparaison, on mentionnera que le taux des particules fines de l'hydroquinone sous forme de poudre est de l'ordre de 20 % en masse ce qui signifie que la teneur des particules fines (ou poussières) est divisée par 10 voire même 20.

Afin de définir la granulométrie du produit de l'invention, on définit également le pourcentage en masse des particules de dimensions inférieures à 2,5 mm c'est-à-dire des particules qui passent à travers un tamis ayant une maille de 2,5 mm.

Cette teneur se situe généralement entre 20 et 40 % en masse.

A titre de comparaison, on mentionnera que 100 % des particules d'hydroquinone sous forme de poudre sont inférieures à 2,5 mm.

Les écailles d'hydroquinone ont une masse volumique qui peut être plus ou moins élevée. La masse volumique apparente (non tassée) des écailles est de préférence entre 0,4 et 0,6 g/cm³, et le plus souvent entre 0,45 et 0,55 g/cm³.

La masse volumique apparente (tassée) des écailles est de préférence, d'au moins 0,5 g/cm³ et se situe encore plus préférentiellement entre 0,5 et 0,8 g/cm³, et le plus souvent entre 0,6 et 0,7 g/cm³.

Les masses volumiques sont mesurées selon le test décrit dans la norme Pharmacopée Européenne [Tome 1, p.256, (2004) 5èmè édition] sur un produit non séché à la seule différence que l'éprouvette de 250 ml est remplacée par une éprouvette de 1 litre.

L'invention réside donc en une nouvelle mise en forme de l'hydroquinone qui bien qu'ayant une forme physique leur permettant de résister à l'attrition, conserve une vitesse de dissolution compatible avec une utilisation postérieure.

Ainsi, la vitesse de dissolution des écailles varie selon l'épaisseur desdites écailles.

Le temps de dissolution dans l'eau d'une quantité d'écailles nécessaire pour obtenir une concentration finale d'hydroquinone de la solution de 4,8 % en masse varie entre 10 et 30 min selon l'épaisseur des écailles.

Ces mesures correspondent à un test qui consiste à mesurer le temps nécessaire pour dissoudre ladite quantité dans l'eau maintenue à la température ambiante (20°C) et sous agitation, par exemple effectuée à l'aide d'un agitateur à hélice à 4 pales inclinées.

Un test similaire est conduit afin de déterminer la vitesse de dissolution des écailles dans l'acide acrylique.

Le test consiste à définir le temps nécessaire pour dissoudre la quantité d'écailles nécessaires pour obtenir une concentration finale d'hydroquinone de 2 % en masse dans l'acide acrylique.

Cette vitesse s'échelonne entre 30 min et 1 h selon l'épaisseur des écailles.

Mesurées dans les mêmes conditions, les vitesses de dissolution de la poudre d'hydroquinone dans l'eau et dans l'acide acrylique sont respectivement de 9 min et 20 min.

Il est à noter que les temps de dissolution de l'hydroquinone mise en forme selon l'invention sont légèrement accrus mais cette augmentation est acceptable par l'utilisateur compte tenu des avantages obtenus par ailleurs.

La structure originale des produits de l'invention est obtenue grâce à un procédé de fabrication parfaitement adapté.

Le procédé de l'invention de préparation des écailles d'hydroquinone est caractérisé par le fait qu'il comprend les étapes suivantes :
- faire fondre si nécessaire la poudre d'hydroquinone,
- déposer l'hydroquinone à l'état liquide en film sur un support en un matériau ou revêtu d'un matériau conducteur de la chaleur,
- faire solidifier l'hydroquinone en amenant le support à la température adéquate,
- récupérer le produit solidifié sous forme d'écailles à l'aide de tout moyen approprié.

Selon un mode préféré de réalisation de l'invention, on élimine au préalable l'oxygène de l'enceinte dans laquelle est effectuée l'opération de mise en forme.

Ainsi, la mise en forme de l'hydroquinone à l'état liquide est effectuée dans une atmosphère débarrassée d'oxygène. Selon un mode d'exécution de l'invention, on établit dans l'enceinte une atmosphère de gaz inertes. On peut faire appel à un gaz rare, de préférence l'argon mais généralement on préfère utiliser l'azote en raison de son coût plus faible.

Une fois l'atmosphère inerte établie, on dépose l'hydroquinone à l'état liquide, en film sur un support approprié.

On peut envisager d'alimenter directement l'hydroquinone à l'état liquide provenant d'une ligne de fabrication.

Il est également possible de prévoir une étape de procédé de l'invention qui consiste à faire fondre l'hydroquinone. A cet effet, on chauffe le produit à sa température de fusion. De préférence, on porte le produit à une température légèrement supérieure à sa température de fusion de 172,5°C, de préférence supérieure à au plus 10°C par rapport à son point de fusion. La température à laquelle est portée celle-ci, est choisie entre 178°C et 185°C.

Cette opération est effectuée, généralement sous agitation.

L'opération peut être effectuée dans une cuve agitée et chauffée. Le chauffage est avantageusement réalisé par circulation de vapeur d'eau ou d'un fluide caloporteur approprié dans la double enveloppe.

Comme fluides caloporteurs convenant à l'invention, on peut mentionner notamment les esters lourds d'acides carboxyliques (par exemple, le phtalate d'octyle), les éthers aromatiques comme l'oxyde de biphényle et/ou l'oxyde de benzyle, le diphényle, les terphényles, les autres polyphényles éventuellement partiellement hydrogénés, les huiles paraffiniques et/ou naphténiques, résidus de distillation du pétrole, les huiles silicones etc...

Conformément au procédé de l'invention, l'hydroquinone à l'état liquide est déposée en film sur un support en un matériau ou revêtu d'un matériau conducteur de la chaleur.

Pour le choix du matériau, on fait appel à tout matériau ne réagissant pas avec l'hydroquinone.

Par ailleurs, comme ce matériau a la propriété de conduire la chaleur, on choisit avantageusement un métal ayant une conductibilité thermique d'au moins 10 W/m.K, de préférence entre 15 et 400 W/m.K. Il est à noter que la borne supérieure ne présente aucun caractère critique.

Comme exemples de matériaux répondant aux caractéristiques précitées et convenant tout à fait bien à la mise en oeuvre du procédé de l'invention, on peut mentionner, entre autres, les aciers inoxydables.

On choisit avantageusement les aciers inoxydables, tels que les aciers austénitiques et plus particulièrement les aciers inoxydables 304, 304 L, 316 ou 316 L.

On met en oeuvre un acier ayant au plus 22 % en masse de nickel, le plus souvent compris entre 6 et 20 %, de préférence compris entre 8 et 14 %.

Les aciers 304 et 304 L ont une teneur en nickel variant entre 8 et 12 % et les aciers 316 et 316 L ont une teneur en nickel variant entre 10 et 14 %.

De tels aciers sont utilisés couramment dans l'industrie.

Pour la définition des aciers austénitiques, on peut se référer à l'ouvrage de Robert H. Perry et al, [Perry's Chemical Engineers' Handbook, Sixth Edition (1984), page 23-44].

Le procédé de l'invention est conduit au moyen d'un dispositif permettant la solidification de l'hydroquinone à l'état liquide sur une surface refroidie consistant ou revêtu d'un matériau conducteur qui peut être sous forme d'une bande transporteuse, d'un ou de plateau(x) tournant(s) ou bien d'un cylindre en rotation.

Le dimensionnement de l'appareillage relève de la compétence de l'Homme du Métier.

Les caractéristiques de la bande transporteuse peuvent varier largement.

Ainsi, la longueur peut aller par exemple entre 50 cm et 2 m, la largeur entre 1 et 5 m. Pour ce qui est de la vitesse de défilement de la bande, elle peut varier avantageusement entre 1 m/min et 20 m/min.

L'épaisseur des écailles est déterminée en contrôlant la vitesse d'alimentation de l'hydroquinone et la vitesse de défilement de la bande.

Pour ce qui est des plateaux tournants, leur diamètre se situe le plus souvent entre 150 et 400 mm.

Leur vitesse de rotation est choisie préférentiellement entre 5 et 50 tours/min.

Selon une première variante de l'invention, l'hydroquinone à l'état liquide est déposée sur une bande ou sur un ou des plateaux tournants par pulvérisation par l'intermédiaire d'une buse et plus couramment par un système à débordement comprenant une auge d'alimentation remplie en hydroquinone à l'état liquide de manière continue de façon à faire déborder l'hydroquinone qui tombe par gravité sur la bande ou le(s) plateau(x).

L'hydroquinone est solidifiée par refroidissement à une température avantageusement comprise entre 20°C et 80°C.

Le plus souvent, le refroidissement est assuré par pulvérisation d'eau froide sur la face interne de la bande qui n'est pas couverte par l'hydroquinone.

Quant au(x) plateau(x) tournant(s), ils sont généralement constitués d'une double enveloppe dans laquelle circule un liquide de refroidissement, de préférence de l'eau introduite à la température adéquate.

On récupère l'hydroquinone sous forme d'écailles grâce à un couteau racleur.

Selon une autre variante du procédé qui est préférée, l'hydroquinone à l'état liquide est déposée sur un cylindre en rotation.

Le cylindre a des dimensions qui peuvent varier largement.

Ainsi, le diamètre peut aller de 0,15 à 2,5 m, de préférence de 1 à 1,5 m et la longueur peut varier par exemple entre 0,25 à 5 m, de préférence 0,5 à 2 m.

L'alimentation du cylindre peut être faite de multiples manières.

Le cylindre étant placé par exemple dans 1 à 10 cm d'hydroquinone à l'état liquide placée dans une auge d'alimentation, le dépôt sur le cylindre s'effectue par trempage.

Le cylindre tourne et entraîne une couche mince de produit qui se solidifie sur le cylindre par refroidissement.

Le cylindre tourne à une vitesse qui est choisie en fonction de l'épaisseur des écailles souhaitée et la température de l'alimentation.

La couche sera d'autant plus mince que la vitesse de rotation est élevée.

L'alimentation de l'hydroquinone à l'état liquide peut se faire sur le cylindre par l'intermédiaire d'un rouleau applicateur, lui-même alimenté en hydroquinone à l'état liquide.

L'alimentation peut être également faite sur le cylindre par coulée par gravité ou par l'intermédiaire d'une pompe.

Le cylindre est refroidi par circulation d'eau dans une double enveloppe ou par pulvérisation d'eau à l'intérieur du cylindre.

Selon une caractéristique du procédé de l'invention, le cylindre est maintenu de préférence à une température comprise entre 20°C et 80°C et plus préférentiellement entre 30°C et 80°C.

La vitesse de rotation du cylindre varie avantageusement entre 0,5 et 20 tours/min, de préférence entre 3 et 6 tours/min.

L'hydroquinone est maintenue suffisamment longtemps sur le cylindre afin qu'elle se solidifie.

Ensuite, on récupère l'hydroquinone mise en forme à l'aide de tout moyen approprié et plus particulièrement à l'aide d'un couteau qui racle le cylindre et décolle la couche de produit qui est récupéré par tout moyen connu par exemple, par gravité dans un bac de récupération.

Ainsi, un mode de réalisation préféré du procédé de l'invention de préparation des écailles d'hydroquinone comprend les étapes suivantes :
- débarrasser l'oxygène de l'enceinte dans laquelle est effectuée l'opération de mise en forme,
- faire fondre si nécessaire la poudre d'hydroquinone,
- déposer l'hydroquinone à l'état liquide en film sur un cylindre maintenu à une température comprise entre 20°C et 80°C,
- maintenir l'hydroquinone suffisamment longtemps sur le cylindre afin qu'elle se solidifie.
- récupérer le produit solidifié à l'aide de tout moyen approprié.

On obtient l'hydroquinone sous forme d'écailles répondant aux caractéristiques donnée ci-dessus.

L'invention n'exclut pas une étape supplémentaire permettant de calibrer le produit obtenu.

Ainsi, les écailles peuvent être introduites par exemple dans un granulateur à couteaux ou à barres permettant de réduire la taille des particules afin d'avoir une répartition plus homogène dans les trois dimensions et obtenir ainsi de l'hydroquinone sous forme de particules isotropes.

Par « particules isotropes », on entend des particules avec trois dimensions équivalentes.

Les particules obtenues s'approchent de la forme cubique et présentent un côté pouvant varier entre 400 et 1500 µm, de préférence entre 500 et 750 µm.

Ainsi, les écailles peuvent être utilisées comme produit intermédiaire pour fabriquer de l'hydroquinone sous forme de particules isotropes.

L'hydroquinone ainsi obtenue présente une densité accrue.

Les exemples qui suivent, illustrent l'invention.

### EXEMPLES

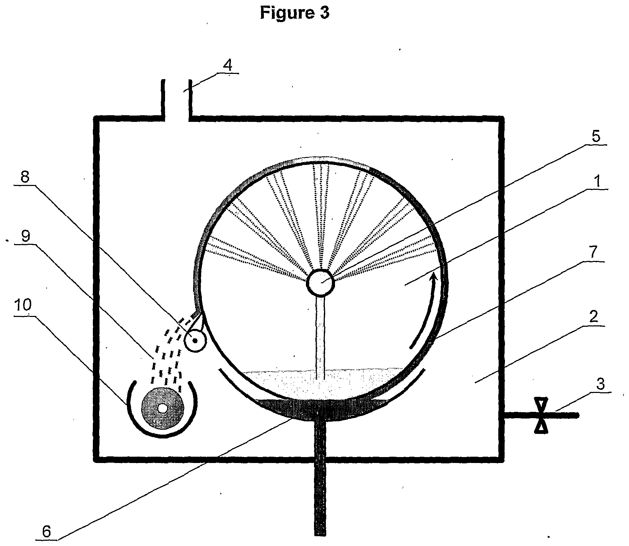

Le procédé de l'invention peut être mis en oeuvre dans l'appareillage décrit ci-dessous et schématisé par la figure 3.

Les écailles d'hydroquinone sont obtenues par solidification d'hydroquinone à l'état liquide sur un cylindre 1 en acier inoxydable (316) en rotation dans une enceinte 2 dans laquelle est établie une atmosphère appauvrie en oxygène par l'introduction d'azote 3. Le gaz chargé en vapeurs d'hydroquinone est évacué de l'enceinte en direction d'un dispositif de traitement des gaz 4.

La température du cylindre est régulée par l'intermédiaire d'une pulvérisation d'eau sur sa face interne 5. Il n'y a pas de contact direct entre l'eau de refroidissement et le produit.

L'hydroquinone à l'état liquide est introduite dans un bac d'alimentation 6 dont la température est régulée par une double enveloppe dans laquelle circule un fluide caloporteur. Le cylindre trempe dans l'hydroquinone en fusion et, du fait de sa rotation, entraîne à sa surface externe un film de produit fondu 7.

Au contact du métal froid, ce film de produit se solidifie progressivement pour arriver solide au niveau d'un couteau raclant 8 qui le décolle du cylindre sous forme d'écailles 9.

Les écailles ainsi obtenues sont collectées dans l'auge d'une vis de transport 10 qui les extrait de l'enceinte.

Les principaux paramètres influant sur la productivité et sur l'épaisseur des écailles produites sont :
- la vitesse de rotation du cylindre, V,
- la température de l'eau de refroidissement, Te,
- la hauteur d'immersion du cylindre dans le produit en fusion, H,
- la température du produit fondu, Tp.

A titre d'exemples, avec un cylindre de 0,75 m² de surface (longueur = 0,48 m - diamètre = 0,50 m), on obtient les résultats récapitulés dans le tableau (I) ci-dessous.

On donne également dans le tableau (I), les caractéristiques physico-chimiques des écailles obtenues.

**Tableau (I)**

| N° exemple | 1 | 2 | 3 | 4 | 5 |
|---|---|---|---|---|---|
| V (tours/minute) | 2 | 4 | 4 | 6 | 10 |
| Te (°C) | 60 | 80 | 40 | 60 | 40 |
| H (mm) | 25 | 25 | 25 | 40 | 40 |
| Tp (°C) | 180 | 180 | 180 | 185 | 185 |
| Productivité (kg/h) | 98 | 126 | 146 | 181 | 281 |
| Epaisseur des écailles (mm) | 0,95 | 0,65 | 0,75 | 0,65 | 0,60 |
| % massique de particules passant à travers un tamis de maille de 100 µm | 0,8% | 0,7% | 0,9% | 0,8% | 0,9% |
| Masse volumique apparente non tassée (g/cm³) | 0,52 | 0,47 | 0,48 | 0,46 | 0,53 |
| Masse volumique apparente tassée (g/cm³) | 0,70 | 0,65 | 0,67 | 0,61 | 0,66 |
| Temps de dissolution dans l'eau à 20°C (obtention d'une solution à 4,8% en masse) | 21 min | 15 min | 18 min | 14 min | 14 min |
| Temps de dissolution dans l'acide acrylique à 20°C (obtention d'une solution à 2,0% en masse) | 45 min | 30 min | 35 min | 30 min | 30 min |

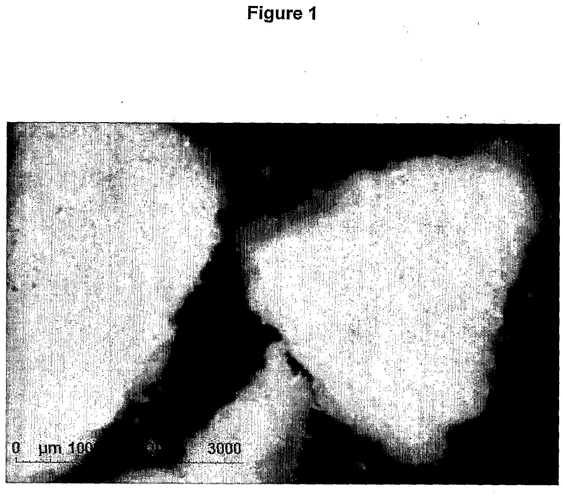

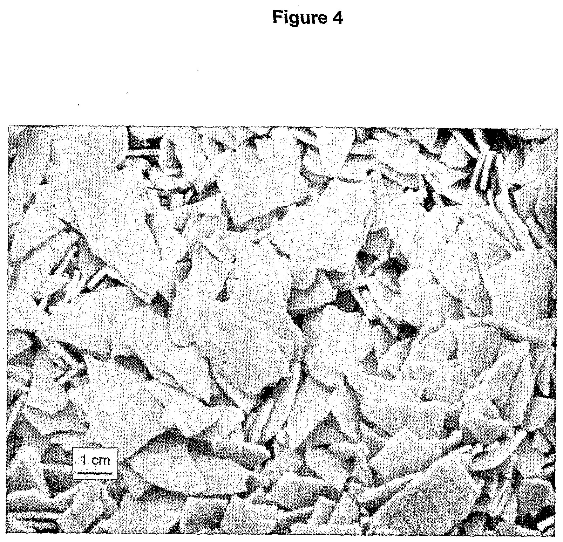

La photographie de la figure 1 illustre la morphologie du produit obtenu selon l'exemple 5. Une vue d'ensemble du produit de l'exemple 5 est donnée par la figure 4.

## Revendications

1. Nouvelle forme d'hydroquinone **caractérisée par le fait qu'**elle se présente sous l'aspect d'écailles, particules massives qui ont une forme plaquettaire de longueur variant entre 0,5 et 6 cm, de largeur variant entre 0,5 et 3 cm, d'épaisseur variant entre 400 µm et 1500 µm, présentant une masse volumique apparente (non tassée) d'au moins 0,4 g/cm³ et dont le pourcentage en masse de particules de dimension inférieure à 100 µm est inférieur à 3%.

2. Nouvelle forme d'hydroquinone selon la revendication 1 **caractérisée par le fait que** les écailles ont une longueur variant entre 1 et 3 cm.

3. Nouvelle forme d'hydroquinone selon l'une des revendications 1 et 2 **caractérisée par le fait que** les écailles ont une largeur variant entre 0,5 et 1,5 cm.

4. Nouvelle forme d'hydroquinone selon l'une des revendications 1 à 3 **caractérisée par le fait que** les écailles ont une épaisseur variant entre 500 µm et 750 µm.

5. Nouvelle forme d'hydroquinone selon l'une des revendications 1 à 4 **caractérisée par le fait que** le pourcentage en masse de particules de dimension inférieure à 100 µm est compris entre 0,7 et 1,5 % en masse.

6. Nouvelle forme d'hydroquinone selon l'une des revendications 1 à 5 **caractérisée par le fait que** le pourcentage en masse de particules de dimension inférieur à 2,5 mm se situe entre 20 et 40 % en masse.

7. Nouvelle forme d'hydroquinone selon l'une des revendications 1 à 6 **caractérisée par le fait qu'**elle présente une masse volumique apparente (non tassée) des écailles se situant entre 0,4 et 0,6 g/cm³.

8. Nouvelle forme d'hydroquinone selon l'une des revendications 1 à 7 **caractérisée par le fait qu'**elle présente une masse volumique apparente (tassée) des écailles d'au moins 0,5 g/cm³.

9. Procédé de préparation des écailles d'hydroquinone décrites dans l'une des revendications 1 à 8 **caractérisé par le fait qu'**il comprend les étapes suivantes :
- faire fondre si nécessaire la poudre d'hydroquinone,
- déposer l'hydroquinone à l'état liquide en film sur un support en un matériau ou revêtu d'un matériau conducteur de la chaleur,
- faire solidifier l'hydroquinone en amenant le support à la température adéquate,
- récupérer le produit solidifié sous forme d'écailles à l'aide de tout moyen approprié.

10. Procédé selon la revendication 9 **caractérisé par le fait que** l'on établit dans l'enceinte où l'hydroquinone est mise en forme, une atmosphère de gaz inertes.

11. Procédé selon l'une des revendications 9 ou 10 **caractérisé par le fait que** le matériau sur lequel est déposé l'hydroquinone a une conductibilité thermique d'au moins 10 W/m.K.

12. Procédé selon la revendication 11 **caractérisé par le fait que** le support est ou est revêtu d'un acier inoxydable.

13. Procédé selon l'une des revendications 9 à 12 **caractérisé par le fait que** l'hydroquinone à l'état liquide est déposée sur une surface refroidie consistant ou revêtu d'un matériau conducteur qui peut être sous forme d'une bande transporteuse, d'un ou de plateau(x) tournant(s) ou bien d'un cylindre en rotation.

14. Procédé selon la revendication 13 **caractérisé par le fait que** le cylindre est placé par exemple dans 1 à 10 cm d'hydroquinone à l'état liquide placée dans une auge d'alimentation, le dépôt sur le cylindre s'effectue par trempage.

15. Procédé selon la revendication 13 **caractérisé par le fait que** l'alimentation de l'hydroquinone à l'état liquide peut se faire sur le cylindre par l'intermédiaire d'un rouleau applicateur, lui-même alimenté en hydroquinone à l'état liquide ou par coulée par gravité ou par l'intermédiaire d'une pompe.

16. Procédé selon l'une des revendications 13 à 15 **caractérisé par le fait que** la vitesse de rotation du cylindre varie entre 0,5 et 20 tours/min.

17. Procédé selon l'une des revendications 13 à 16 **caractérisé par le fait que** le cylindre est maintenu une température comprise entre 20°C et 80°C.

18. Procédé selon l'une des revendications 13 à 17 **caractérisé par le fait que** le cylindre est refroidi par circulation d'eau dans une double enveloppe ou par pulvérisation d'eau à l'intérieur du cylindre.

19. Procédé selon l'une des revendications 13 à 18 **caractérisé par le fait que** l'on récupère l'hydroquinone mise en forme à l'aide d'un couteau qui racle le cylindre et décolle la couche de produit qui est récupéré.

20. Procédé selon l'une des revendications 13 à 19 **caractérisé par le fait qu'**il comprend les étapes suivantes :
- débarrasser l'oxygène de l'enceinte dans laquelle est effectuée l'opération de mise en forme,
- faire fondre si nécessaire la poudre d'hydroquinone,
- déposer l'hydroquinone à l'état liquide en film sur un cylindre maintenu à une température comprise entre 20°C et 80°C,
- maintenir l'hydroquinone suffisamment longtemps sur le cylindre afin qu'elle se solidifie.
- récupérer le produit solidifié à l'aide de tout moyen approprié.

21. Procédé selon l'une des revendications 1 à 20 **caractérisé par le fait que** l'on soumet les écailles obtenues à une opération de calibrage permettant d'obtenir une répartition plus homogène de la taille des particules.

22. Procédé selon la revendication 21 **caractérisé par le fait que** l'opération est effectuée dans un granulateur à couteaux ou à barres.

23. Utilisation des écailles d'hydroquinone décrites dans l'une des revendications 1 à 8 comme produit intermédiaire pour fabriquer de l'hydroquinone sous forme de particules isotropes.

## Patentansprüche

1. Neuartige Form von Hydrochinon, **dadurch gekennzeichnet, dass** es in Form von Flocken, massiven Teilchen, die eine Plättchenform von einer Länge, die zwischen 0,5 und 6 cm variiert, von einer Breite, die zwischen 0,5 und 3 cm variiert, von einer Dicke, die zwischen 400 µm und 1.500 µm variiert, aufweisen, auftritt, die eine Schüttdichte (die nicht verdichtet ist) von mindestens 0,4 g/cm³ aufweisen und deren Massenanteil an Teilchen mit einer Größe von weniger als 100 µm weniger als 3 % beträgt.

2. Neuartige Form von Hydrochinon nach Anspruch 1, **dadurch gekennzeichnet, dass** die Flocken eine Länge aufweisen, die zwischen 1 und 3 cm variiert.

3. Neuartige Form von Hydrochinon nach einem der Ansprüche 1 und 2, **dadurch gekennzeichnet, dass** die Flocken eine Breite aufweisen, die zwischen 0,5 und 1,5 cm variiert.

4. Neuartige Form von Hydrochinon nach einem der Ansprüche 1 bis 3, **dadurch gekennzeichnet, dass** die Flocken eine Dicke aufweisen, die zwischen 500 µm und 750 µm variiert.

5. Neuartige Form von Hydrochinon nach einem der Ansprüche 1 bis 4, **dadurch gekennzeichnet, dass** der Massenanteil an Teilchen mit einer Größe von weniger als 100 µm zwischen 0,7 und 1,5 Massen% beträgt.

6. Neuartige Form von Hydrochinon nach einem der Ansprüche 1 bis 5, **dadurch gekennzeichnet, dass** der Massenanteil an Teilchen mit einer Größe von weniger als 2,5 mm zwischen 20 und 40 Massen% beträgt.

7. Neuartige Form von Hydrochinon nach einem der Ansprüche 1 bis 6, **dadurch gekennzeichnet, dass** es eine Schüttdichte (die nicht verdichtet ist) der Schuppen aufweist, die zwischen 0,4 und 0,6 g/cm³ beträgt.

8. Neuartige Form von Hydrochinon nach einem der Ansprüche 1 bis 7, **dadurch gekennzeichnet, dass** es eine Schüttdichte (die nicht verdichtet ist) der Flocken von mindestens 0,5 g/cm³ aufweist.

9. Verfahren zur Herstellung von Hydrochinon-Flocken, die in einem der Ansprüche 1 bis 8 beschrieben werden, **dadurch gekennzeichnet, dass** es die folgenden Schritte aufweist:
- erforderlichenfalls das Hydrochinon-Pulver schmelzen,
- Aufbringen des Hydrochinons in flüssigem Zustand als einen Film auf einen Träger aus einem wärmeleitendem Material oder der mit einem wärmeleitenden Material beschichtet ist,
- Verfestigen des Hydrochinons, indem der Träger auf die geeignete Temperatur gebracht wird,
- Gewinnen des verfestigten Produktes in Form von Flocken mit Hilfe von jedem geeigneten Mittel.

10. Verfahren nach Anspruch 9, **dadurch gekennzeichnet, dass** in dem Behälter, in dem das Hydrochinon in Form gebracht wird, eine Inertgasatmosphäre hergestellt wird.

11. Verfahren nach einem der Ansprüche 9 oder 10, **dadurch gekennzeichnet, dass** das Material, auf das das Hydrochinon aufgebracht wird, eine Wärmeleitfähigkeit von mindestens 10 W/m.K aufweist.

12. Verfahren nach Anspruch 11, **dadurch gekennzeichnet, dass** der Träger aus einem Edelstahl ist oder damit beschichtet ist.

13. Verfahren nach einem der Ansprüche 9 bis 12, **dadurch gekennzeichnet, dass** das Hydrochinon in flüssigem Zustand auf eine gekühlte Oberfläche aufgebracht wird, die aus einem leitenden Material besteht oder damit beschichtet ist, das in Form von einem Transportband, von einem Drehteller (oder von Drehtellern) oder einem rotierenden Zylinder sein kann.

14. Verfahren nach Anspruch 13, **dadurch gekennzeichnet, dass** der Zylinder beispielsweise in 1 bis 10 cm Hydrochinon in flüssigem Zustand angeordnet wird, das in einer Speisemulde angeordnet ist, dass das Auftragen auf den Zylinder durch Eintauchen erfolgt.

15. Verfahren nach Anspruch 13, **dadurch gekennzeichnet, dass** die Zufuhr des Hydrochinons in flüssigem Zustand auf dem Zylinder mittels einer Auftragswalze, die selbst mit Hydrochinon in flüssigem Zustand versorgt wird, oder durch Schwerkraftgießen oder über eine Pumpe erfolgen kann.

16. Verfahren nach einem der Ansprüche 13 bis 15, **dadurch gekennzeichnet, dass** die Rotationsgeschwindigkeit des Zylinders zwischen 0,5 und 20 Umdrehungen/Minute variiert.

17. Verfahren nach einem der Ansprüche 13 bis 16, **dadurch gekennzeichnet, dass** der Zylinder auf einer Temperatur gehalten wird, die zwischen 20 °C und 80 °C beträgt.

18. Verfahren nach einem der Ansprüche 13 bis 17, **dadurch gekennzeichnet, dass** der Zylinder durch Umlaufen von Wasser in einer doppelten Hülle oder durch Sprühen von Wasser ins Innere des Zylinders gekühlt wird.

19. Verfahren nach einem der Ansprüche 13 bis 18, **dadurch gekennzeichnet, dass** das in Form gebrachte Hydrochinon mittels eines Messers gewonnen wird, das den Zylinder abkratzt und die Produktschicht abhebt, die gewonnen wird.

20. Verfahren nach einem der Ansprüche 13 bis 19, **dadurch gekennzeichnet, dass** es den folgenden Schritte aufweist:
- Beseitigen des Sauerstoffes aus dem Behälter, in dem der Vorgang des Informbringens durchgeführt wird,
- erforderlichenfalls das Hydrochinon-Pulver schmelzen,
- Aufbringen des Hydrochinons in flüssigem Zustand als einen Film auf einen Zylinder, der auf einer Temperatur gehalten wird, die zwischen 20 °C und 80 °C beträgt,
- das Hydrochinon ausreichend lange auf dem Zylinder halten, damit es sich verfestigt,
- Gewinnen des verfestigten Produktes mit Hilfe von jedem geeigneten Mittel.

21. Verfahren nach einem der Ansprüche 1 bis 20, **dadurch gekennzeichnet, dass** die erhaltenen Flocken einer Kalibrierungsoperation unterworfen werden, die ermöglicht, eine gleichmäßigere Verteilung der Größe der Teilchen zu erhalten.

22. Verfahren nach Anspruch 21, **dadurch gekennzeichnet, dass** die Operation in einem Messergranulator oder einem Granulator mit Stäben durchgeführt wird.

23. Verwendung von Hydrochinon-Flocken, die in einem der Ansprüche 1 bis 8 beschrieben werden, als Zwischenprodukt, um Hydrochinon in Form von isotropen Teilchen herzustellen.

## Claims

1. New form of hydroquinone, **characterized in that** it is provided under the appearance of flakes, large particles which have a platelet shape with a length varying between 0.5 and 6 cm, with a width varying between 0.5 and 3 cm, with a thickness varying between 400 µm and 1500 µm, exhibiting a (loose) bulk density of at least 0.4 g/cm³ and the percentage by weight of particles with a dimension of less than 100 µm of which is less than 3%.

2. New form of hydroquinone according to Claim 1, **characterized in that** the flakes have a length varying between 1 and 3 cm.

3. New form of hydroquinone according to either of Claims 1 and 2, **characterized in that** the flakes have a width varying between 0.5 and 1.5 cm.

4. New form of hydroquinone according to one of Claims 1 to 3, **characterized in that** the flakes have a thickness varying between 500 µm and 750 µm.

5. New form of hydroquinone according to one of Claims 1 to 4, **characterized in that** the percentage by weight of particles with a dimension of less than 100 µm is between 0.7 and 1.5% by weight.

6. New form of hydroquinone according to one of Claims 1 to 5, **characterized in that** the percentage by weight of particles with a dimension of less than 2.5 mm is between 20 and 40% by weight.

7. New form of hydroquinone according to one of Claims 1 to 6, **characterized in that** it exhibits a (loose) bulk density of the flakes which is between 0.4 and 0.6 g/cm³.

8. New form of hydroquinone according to one of Claims 1 to 7, **characterized in that** it exhibits a (tapped) bulk density of the flakes of at least 0.5 g/cm³.

9. Process for the preparation of the hydroquinone flakes described in one of Claims 1 to 8, **characterized in that** it comprises the following stages:
- if necessary melting the hydroquinone powder,
- depositing the hydroquinone in the liquid state as a film on a support made of a thermally conductive material or coated with a thermally conductive material,
- solidifying the hydroquinone by bringing the support to the appropriate temperature,
- recovering the solidified product in the form of flakes using any appropriate means.

10. Process according to Claim 9, **characterized in that** an atmosphere of inert gases is established in the chamber where the hydroquinone is formed.

11. Process according to either of Claims 9 and 10, **characterized in that** the material on which the hydroquinone is deposited has a thermal conductivity of at least 10 W/m.K.

12. Process according to Claim 11, **characterized in that** the support is or is coated with a stainless steel.

13. Process according to one of Claims 9 to 12, **characterized in that** the hydroquinone in the liquid state is deposited on a cooled surface consisting of a conductive material or coated with a conductive material which can be in the form of a conveyor belt, of one or more turntable (s) or else of a rotating cylinder.

14. Process according to Claim 13, **characterized in that** the cylinder is placed, for example, in 1 to 10 cm of hydroquinone in the liquid state placed in a feed trough and the deposition on the cylinder is carried out by dipping.

15. Process according to Claim 13, **characterized in that** the feeding of the hydroquinone in the liquid state can be carried out on the cylinder via an applicator roll, itself fed with hydroquinone in the liquid state, or by pouring by gravity or via a pump.

16. Process according to one of Claims 13 to 15, **characterized in that** the rotational speed of the cylinder varies between 0.5 and 20 revolutions/min.

17. Process according to one of Claims 13 to 16, **characterized in that** the cylinder is maintained at a temperature of between 20°C and 80°C.

18. Process according to one of Claims 13 to 17, **characterized in that** the cylinder is cooled by circulating water in a jacket or by spraying water inside the cylinder.

19. Process according to one of Claims 13 to 18, **characterized in that** the formed hydroquinone is recovered using a blade which scrapes the cylinder and detaches the layer of product, which is recovered.

20. Process according to one of Claims 13 to 19, **characterized in that** it comprises the following stages:
- freeing from oxygen the chamber in which the forming operation is carried out,
- if necessary melting the hydroquinone powder,
- depositing the hydroquinone in the liquid state as a film on a cylinder maintained at a temperature of between 20°C and 80°C,
- maintaining the hydroquinone on the cylinder for a sufficient length of time for it to solidify,
- recovering the solidified product using any appropriate means.

21. Process according to one of Claims 1 to 20, **characterized in that** the flakes obtained are subjected to a grading operation which makes it possible to obtain a more homogeneous distribution in the size of the particles.

22. Process according to Claim 21, **characterized in that** the operation is carried out in a blade or bar granulator.

23. Use of the hydroquinone flakes described in one of Claims 1 to 8 as intermediate, in order to manufacture hydroquinone in the form of isotropic particles.
